(19) Europäisches Patentamt
European Patent Office
Office européen des brevets

(11) Publication number: **0 131 998**
**B1**

(12) # EUROPEAN PATENT SPECIFICATION

(45) Date of publication of patent specification: **01.07.87**

(51) Int. Cl.[4]: **C 07 C 29/32,** C 07 C 67/36, C 07 C 31/08, C 07 C 69/14

(21) Application number: **84200964.9**

(22) Date of filing: **03.07.84**

(54) **Process for the preparation of ethanol and/or ethyl acetate.**

(30) Priority: **06.07.83 NL 8302396**

(43) Date of publication of application:
**23.01.85 Bulletin 85/04**

(45) Publication of the grant of the patent:
**01.07.87 Bulletin 87/27**

(84) Designated Contracting States:
**BE DE FR GB IT NL**

(56) References cited:
**EP-A-0 031 606**
**EP-A-0 051 859**
**EP-A-0 085 204**
**US-A-4 301 312**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Drent, Eit**
**Badhuisweg 3**
**NL-1031 CM Amsterdam (NL)**

(74) Representative: **Aalbers, Onno et al**
**P.O. Box 302**
**NL-2501 CH The Hague (NL)**

**EP 0 131 998 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to a process for the preparation of ethanol and/or ethyl acetate by reaction of methanol with carbon monoxide or a mixture of carbon monoxide and hydrogen in the presence of a catalyst system containing a ruthenium compound and an organic nitrogen compound.

There is a lively interest in processes by which, starting from synthesis gas ($CO+H_2$) optionally obtained from coal or natural gas, ethanol can be produced on a technical scale and in an economically justifiable manner. Since processes for producing methanol from carbon monoxide and hydrogen have already been developed on a commercial scale, it would seem attractive to have the preparation of ethanol from synthesis gas be realised via methanol. Although the conversion of methanol into ethanol by reaction with synthesis gas has been studied extensively, as is seen for instance from a review article by H. Bahrmann, W. Lipps and B. Cornils in Chemiker Zeitung *106*, pp. 249—258 (1982), the known methods still have various drawbacks.

A process of the type to which the invention relates is described in US—A—4,301,312. By this known process ethanol is produced according to the reaction equation

$$CH_3OH+2CO+H_2 \rightarrow C_2H_5OH+CO_2. \qquad I$$

This known process is carried out in the presence of a tertiary amine, for example trimethylamine and N-methylpyrrolidine. However, a drawback of this known process is that in order to achieve optimum results it is necessary to use a very high pressure, i.e. from 101 to 404 bar, the experiments described in the examples being carried out at 303 or 380 bar. This process aims at reducing formation of water to a minimum.

EP—A—51,859 describes a process for the preparation of ethanol and propanol by reaction of methanol with carbon monoxide and hydrogen in the presence of a cobalt compound, a ruthenium compound, iodine or an iodide, an organic phosphine or phosphite, and water. This known process also has the drawback that it is necessary to apply a very high pressure, i.e. from 200 to 600 bar, the experiments described in the examples being carried out at 280 or 550 bar. When carrying out the process on a technical scale this is rather unattractive for economic reasons, for instance because unconverted synthesis gas, methanol and some by-products have to be recirculated to the high-pressure-reactor. Besides, the number of by-products formed in this process is very large and hinders the production of ethanol. On top of this some of the by-products, such as 1,1-dimethoxyethane, are not very suitable for recirculation to the reactor. Another serious drawback is that during the cobalt-compound catalysed reaction of methanol with synthesis gas water is formed. According to the reaction equation

$$CH_3OH+CO+2H_2 \rightarrow C_2H_5OH+H_2O \qquad II$$

—mentioned in, for instance, UK patent application No. 2,036,739 A—one molecule of water is formed per molecule of ethanol. The presence of water in the reaction mixture has an unfavourable influence on catalyst stability and it is the reason why distillate processing of the reaction mixture yields nothing but ethanol-water mixtures.

Publications by M. J. Chen, H. M. Feder and J. W. Rathke in J. Am. Chem. Soc. *104* (1982) 7346—7347 and Journal of Molecular Catalysis *17* (1982) 331—337 show that reaction I is also catalysed by carbonyl compounds and manganese and rhodium, and that the best results are obtained by using an $Mn_2(CO)_{10}$—$Fe(CO)_5$ catalyst. It is further mentioned that the addition of methyl iodide enhances the activity but not the selectivity of the manganese carbonyl compound. However, the Feder et al. method, like the afore-mentioned process in which a cobalt-containing catalyst is used, has the drawback that high pressures (about 300 bar) must necessarily be used.

US patent specification No. 4,370,507 describes a process for the preparation of ethanol by reaction of methanol with carbon monoxide and hydrogen at a low pressure (about 16 bar) in the presence of a heterogeneous catalyst containing rhodium and iron in a reduced state supported on a carrier of aluminium oxide which has been impregnated with a heterocyclic amine, in particular pyridine. However, this process has the drawback that, among other things, water and large amounts of hydrocarbons are formed as by-products. Moreover from the test results mentioned in this patent specification it may be concluded that a considerable portion of the methanol decomposes to form a mixture of CO and $H_2$.

A process has now been found in which by reaction of methanol with carbon monoxide or carbon monoxide and hydrogen, ethanol can be prepared at a low pressure with good selectivity, without water being produced as by-product. As second product ethyl acetate is formed, a compound which finds application on a large scale as solvent. If desired, ethyl acetate can also be obtained as main product, and therefore the invention also relates to the direct preparation of ethyl acetate from methanol and synthesis gas.

The invention relates to a process for the preparation of ethanol and/or ethyl acetate by reaction of methanol with carbon monoxide or a mixture of carbon monoxide and hydrogen in the presence of a catalyst system containing a ruthenium compound and an organic nitrogen compound, characterized in that an iodide and/or bromide of an alkali metal or an alkaline earth metal is present in the reaction mixture

and that the organic nitrogen compound comprises an aromatic heterocyclic amine or an oxide thereof or contains a group

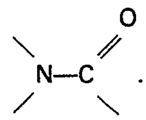 .

The catalyst system in addition to the ruthenium compound preferably also contains a rhodium compound. The use of a ruthenium compound in addition to a rhodium compound and in combination with an iodide and/or bromide as a catalytic system is as such known from EP—A—31,606, but this known process involves a different reaction, viz.:

$$2CH_3COOCH_3+2CO+2H_2 \rightarrow CH_3COOC_2H_5+2CH_3COOH.$$

The formation of ethanol and ethyl acetate occurs according to the following overall reaction equations:

$$CH_3OH+2CO+H_2 \rightarrow C_2H_5OH+CO_2 \qquad \text{III}$$

$$2CH_3OH+4CO \rightarrow CH_3COOC_2H_5+2CO_2. \qquad \text{IV}$$

It is not imperative that the hydrogen used in reaction III should be introduced from outside. As has been stated before, the process according to the invention can be carried out both by using carbon monoxide and by using mixtures of carbon monoxide and hydrogen; the fact is that under the influence of the catalyst system used according to the invention hydrogen is formed in situ according to the reaction equation:

$$CO+H_2O \rightarrow CO_2+H_2. \qquad \text{V}$$

The water used in reaction V may be formed, for instance, by the reaction equation:

$$2CH_3OH+CO \rightarrow CH_3COOCH_3+H_2O. \qquad \text{VI}$$

The by-product methyl acetate formed according to reaction VI can contribute—optionally after isolation from the reaction mixture and recirculation to the reactor—to the formation of additional quantities of ethyl acetate according to the reaction equation:

$$2CH_3COOCH_3+2CO+2H_2 \rightarrow CH_3COOC_2H_5+2CH_3COOH. \qquad \text{VII}$$

From the acetic acid formed methyl acetate can be formed again according to the reaction equation:

$$CH_3COOH+CH_3OH \rightarrow CH_3COOCH_3+H_2O. \qquad \text{VIII}$$

The water released in reaction VIII also decomposes according to reaction equation V to form carbon dioxide and hydrogen.

The by-products formed in the process according to the invention are almost exclusively methyl acetate, methane, carbon dioxide and acetic acid, if any. As stated hereinbefore, methyl acetate and acetic acid can contribute to the formation of ethyl acetate. Of compounds such as acetaldehyde and 1,1-dimethoxyethane, which are often formed as by-products in the preparation of ethanol by reaction of methanol with carbon monoxide and hydrogen not more than traces are found. Water formed during the process decomposes according to reaction V, so that in the end the reaction mixture formed will be free of water.

The choice of the ruthenium compound used in the process according to the invention is not very critical. Suitable for instance are ruthenium compounds such as ruthenium(III) chloride, ruthenium(III) chloride trihydrate, ruthenium(III) bromide, ruthenium(III) nitrate, the ruthenium oxides, organic ruthenium salts, such as ruthenium acetate, ruthenium propionate, ruthenium butyrate and ruthenium naphthenate, carbonyl compounds such for instance as $Ru(CO)_5$, $H_4Ru_4(CO)_{12}$ and $RuCl_3(CO)_3$ and organic complexes such for instance as ruthenium(III) acetyl acetonate.

The choice of the rhodium compound preferred in the catalyst system is also little critical. Suitable are for instance rhodium compounds such as the rhodium oxides $Rh_2O_3$ and $RhO_2$, rhodium(III) chloride, rhodium(III) chloride trihydrate, rhodium(III) bromide and rhodium(III) iodide, rhodium(III) sulphate, organic rhodium salts such as rhodium formate, rhodium acetate, rhodium butryate and rhodium naphthenate, carbonyl compounds such as dirhodium octacarbonyl, tetrarhodium dodecacarbonyl and hexarhodium hexadecacarbonyl and complexes such as dichlorobis-(triphenylphosphine)rhodium, tri-(pyridine)rhodium(III) chloride and rhodiumdicarbonyl acetyl acetonate.

If the catalyst system contains both ruthenium and rhodium, the ratio of the number of gram atoms ruthenium to the number of gram atoms rhodium preferably lies between 50:1 and 1:5, in particular between 20:1 and 1:2.

It is an essential characteristic of the process according to the invention that the reaction is carried out in the presence of an iodide and/or bromide of an alkali or alkaline earth metal. If instead of such an iodide or bromide use is made for instance of methyl iodide or zinc iodide, then the formation of ethers and/or acetic acid will be predominant. However, this does not exclude the possibility of methyl iodide or bromide or any other iodine or bromine compound being added to the reaction mixture, for instance in order to supplement iodine or bromine discharged from the reactor during continuous operation of the process according to the invention, for instance as methyl iodide or methyl bromide formed from the alkali metal iodide or bromide. Suitable iodides or bromides of alkali or alkaline earth metals are LiI, NaI, KI, RbI, CsI, LiBr, NaBr, KBr, RbBr, CsBr, $MgI_2$, $CaI_2$, $SrI_2$, $BaI_2$, $MgBr_2$, $CaBr_2$, $SrBr_2$ and $BaBr_2$. Iodides are preferred to bromides. Lithium iodide and sodium iodide are iodides used by preference. The ratio of the number of gram atoms I plus Br to the number of gram atoms Ru plus Rh preferably lies between 3:1 and 200:1, in particular between 4:1 and 150:1.

It is further an essential characteristic of the process of the invention that a nitrogen compound of one of the types indicated hereinbefore is present in the reaction mixture. As distinct from the amines used in the examples of US patent specification No. 4,301,312, trimethylamine and N-methylpyrrolidine, these nitrogen compounds are weak bases. Strong nitrogen bases act very unfavourably in the process according to the invention.

The aromatic heterocyclic amine which can be used according to the invention preferably contains a heterocyclic six-membered ring. Therefore preference is given to heterocyclic amines such for instance as pyridine, chinoline and isochinoline, which compounds may optionally contain one or more substituents, for instance hydrocarbyl groups, in particular alkyl groups having 1—6 carbon atoms or halogen atoms, and the oxides thereof. Pyridine and pyridine oxide are excellently suitable. Specific examples of suitable substituted aromatic heterocyclic amines are alpha-, beta- and gamma-picoline, 4,4′-trimethylene-bipyridine, 2,2′-bipyridine, 2-ethyl-4-chloropyridine, 3,5-dimethylpyridine, 2-cyclohexylpyridine and chinaldine. The picolines and their oxides are preferred organic nitrogen compounds with a substituted aromatic heterocyclic ring.

The organic nitrogen compound containing a group

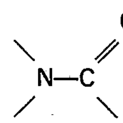

may be an amide, a carbamate, urea or a urea derivate. Suitable amides have the formula:

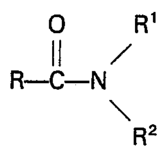 , IX

wherein R represents a hydrogen atom or an alkyl, cycloalkyl, aryl, alkaryl or aralkyl group preferably having not more than 10 carbon atoms, $R^1$ and $R^2$ independently represent a hydrogen atom or an alkyl, aryl, alkaryl or aralkyl group which may contain a

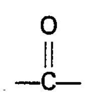

group and preferably has not more than 10 carbon atoms, or $R^1$ and $R^2$, together with the nitrogen atom to which they are bound, form a cyclic group which preferably has not more than 5 carbon atoms and which may contain one or more nitrogen or oxygen atoms, or one of the groups $R^1$ and $R^2$ together with the nitrogen atom and R, forms a cyclic group preferably having not more than 5 carbon atoms. Specific examples of such amides are formamide, acetamide, N-methylacetamide, N-ethylacetamide, N,N-dimethylformamide, N,N-diethylformamide, N,N-dimethylacetamide, N,N-diethylacetamide, N-methyl-N-ethylacetamide, N,N-dimethylcyclohexylcarboxamide, N,N-dimethylbenzamide, N-phenylacetamide, N,N-diphenylacetamide, N-methyl-N-phenylacetamide, N-formylmorpholine, N-acetylmorpholine, N-formylpiperidine, N-acetylpiperidine and N-acetyl-N′-methylpiperazine. Especially suitable amides are N,N-dimethylacetamide and N-methylpyrrolidone. Formula IX also includes diamides and triamides such as diacetamide, triacetamide, dibenzamide, tribenzamide and N-methyldibenzamide, and imides such as succinimide, 1,2-cyclohexane dicarboximide and N-phenylphthalimide.

Suitable carbamates for instance have the formula:

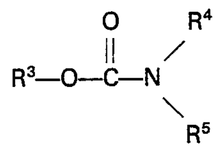

, X

wherein $R^3$ represents an alkyl, aryl, aralkyl or alkaryl group preferably having not more than 10 carbon atoms and $R^4$ and $R^5$ independently represent a hydrogen atom or an alkyl, aryl, aralkyl or alkaryl group preferably having not more than 10 carbon atoms. Specific examples of such carbamates are methyl carbamate, ethyl carbamate, phenyl carbamate, N-methylmethyl carbamate, N-ethylmethyl carbamate, N-phenylmethyl carbamate, N-methylethyl carbamate, N-ethylethyl carbamate, N-phenylethyl carbamate, N-methylphenyl carbamate, N-ethylphenyl carbamate, N-phenylphenyl carbamate N-N-dimethylmethyl carbamate, N,N-diethylmethylethyl carbamate, N,N-diphenylmethyl carbamate, N,N-dimethylethyl carbamate, N,N-diethylethyl carbamate, N,N-diphenylethyl carbamate, N,N-dimethylphenyl carbamate, N,N-diethylphenyl carbamate, N-diphenylphenyl carbamate, N-methyl-N-ethylmethyl carbamate and N-methyl-N-ethylethyl carbamate.

Urea and its derivatives have the formula:

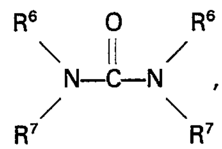

, XI

wherein $R^6$ and $R^7$ independently represent a hydrogen atom or an alkyl, aryl, aralkyl or alkaryl group preferably having not more than 10 carbon atoms. Specific examples of such compounds are urea, 1,3-dimethylurea, 1,3-diethylurea, 1,3-diphenylurea, 1,1-dimethylurea, 1,1-diphenylurea and 1,1,3,3-tetramethylurea.

The ratio of the number of moles of the aromatic heterocyclic amine, the oxide thereof or the organic nitrogen compound containing a group

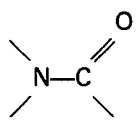

to the number of gram atoms Ru+Rh generally lies between 5:1 and 200:1. In the case of the organic nitrogen compounds containing a group

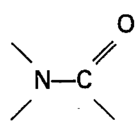

this ratio preferably lies between 30:1 and 200:1 and in the case of the aromatic heterocyclic amines and the oxides thereof this ratio preferably lies between 5:1 and 50:1. However, the use of higher or lower ratios is not precluded.

The process of the invention is preferably carried out at a temperature between 150 and 250°C, in particular between 160 and 220°C. The overall pressure preferably lies between 40 and 100 bar. Higher pressures, for instance of up to 1000 bar may be used if desired, but generally they are undesirable for technical and economic reasons.

The carbon monoxide or the mixture of carbon monoxide and hydrogen may optionally be mixed with other gases such for instance as carbon dioxide, methane, nitrogen or noble gases. If a mixture of carbon monoxide and hydrogen is used, the carbon monoxide to hydrogen molar ratio preferably is at least 1:1, in particular at least 2:1.

The process of the invention may be carried out under anhydrous conditions. However, since any water present or formed is converted into $CO_2$ and $H_2$ by reaction with carbon monoxide according to reaction equation V, there will be no problem if the starting materials contain some water, for instance in the form of crystal water. When, for instance, a carbon monoxide feed with a high CO content and a low hydrogen content is available, it may even be advantageous to add some water to the reaction mixture in the course of the reaction in order to stimulate the formation of hydrogen in situ. When water is added continuously it will of course be possible that the reaction mixture is not entirely anhydrous. Addition of water on the other hand leads to a reduction in the formation of methane.

The reaction may optionally be carried out in the presence of a promoter. Suitably promoters are for instance the oxides of secondary and tertiary phosphines. Examples of suitable phosphine oxides are

trimethylphosphine oxide, diethylphosphine oxide, tri-n-butylphosphine oxide, trioctylphosphine oxide, diphenylphosphine oxide, tri-p-tolylphosphine oxide, tricyclohexylphosphine oxide, diphenylethylphosphine oxide, tri(1-naphthyl)phosphine oxide and tri-4-chlorophenylphosphine oxide. The phosphorus atom of the phosphine oxide may also be part of a heterocyclic system such for instance as in 1-phenylphospholane oxide, 1-phenylphosphorinane oxide and 9-phenyl-9-phosphabicyclo[3-3-1]nonane oxide. Oxides of phosphines containing two or more phosphine groups, such for instance as tetraphenyldiphosphine ethane, can also be used. Preference is given to phosphine oxides of the formula $R^8_3P{=}O$, wherein the groups $R^8$ independently represent alkyl groups of 1—12 carbon atoms or phenyl groups optionally substituted with methyl or ethyl groups. The amounts of phosphine oxide may vary within wide ranges. Suitable quantities lie, for instance, between 1 and 100 mol per gram atom Ru plus Rh, but larger or smaller quantities may also be used.

Another group of promoters are basic compounds of alkali or alkaline earth metals such for instance as the oxides, hydroxides, carbonates, bicarbonates, formates, acetates, and alcoholates of lithium, sodium, potassium, cesium, magnesium, calcium and barium.

The process is carried out in the liquid phase. Generally there is no need to use an additional solvent since methanol, the nitrogen compound and the products formed act sufficiently as solvents. If desired, additional solvents may be used however, tetrahydrothiophene-1,1-dioxide, also referred to as "sulpholane", for instance.

The process can be carried out continuously, semi-continuously or batch-wise. The reaction mixture obtained can be processed by known techniques, such for instance as fractional distillation. The process may further be integrated in existing processes for preparing the starting materials or working up the products obtained.

Example I

Into a magnetically stirred 125 ml Hastelloy C autoclave (Hastelloy is a trade mark) were placed 0.62 mol methanol (25 ml), 0.5 mmol $RuCl_3 \cdot 3H_2O$, 0.25 mmol $RhCl_3 \cdot 3H_2O$, 60 mmol $LiI \cdot 2H_2O$ and 12 mmol pyridine. The autoclave was flushed with carbon monoxide, filled with a mixture of carbon monoxide and hydrogen of a molar ratio of 2:1 and an overall pressure of 60 bar and then heated to a temperature of 190°C. After the reaction time of 15 hours the reaction mixture was analysed by gas-liquid chromatography. Methanol conversion was 15 %mol and selectivities to the formation of ethanol, methyl acetate and methane were 85, 8 and 5 %mol, respectively. Traces of ethyl acetate were detected. The reaction mixture obtained was anhydrous.

Example II

Into a magnetically stirred 125 ml Hastelloy C autoclave were placed 0.62 mol methanol (25 ml), 50 mmol $LiI \cdot 2H_2O$ and the quantities given in Table A of ruthenium compound, rhodium compound, nitrogen compound and promoter. The autoclave was flushed with carbon monoxide, filled with a mixture of carbon monoxide and hydrogen of a molar ratio of 2:1 and an overall pressure of 60 bar, and heated to the temperatures given in the table. During the reaction mixtures of carbon monoxide and hydrogen of the molar ratios given in Table A were introduced into the autoclave at a rate of 9 Nl/h for the periods of time given in Table A, so as to strip volatile components from the reaction mixture. These components were passed through a pressure valve into a condensation unit. The overall pressure in the autoclave was kept at 60 bar. To the mixture of carbon monoxide and hydrogen to be introduced a mixture of methanol (13 %mol), water (47 %mol) and methyl iodide (39 %mol) was added continuously at a rate of 0.4 ml/h. The total reaction time was 15 hours for all the reactions. After termination of the reactions the contents both of the autoclave and of the condensation unit were analysed by gas-liquid chromatography. At each hour of the experiment samples were taken of the effluent gas stream and analysed by gas chromatography for the presence of CO, $H_2$, $CH_4$ and $CO_2$. The conversion of methanol and the selectivities towards the formation of ethanol, methyl acetate and methane are given in Table A.

Example III

Into a magnetically stirred 125 ml Hastelloy C autoclave were placed 0.62 mol methanol (25 ml), 1 mmol $Ru(acac)_3$, 0.25 mmol $Rh(acac)CO_2$, 50 mmol $LiI \cdot 2H_2O$, 10 mmol alpha-picoline and 8 mmol triphenylphosphine oxide. The autoclave was flushed with carbon monoxide, filled up with a mixture of carbon monoxide and hydrogen of molar ratio of 2:1 and an overall pressure of 60 bar, and then heated to a temperature of 190°C. During the reaction a mixture of carbon monoxide and hydrogen was introduced into the autoclave at a rate of 9 Nl/h, stripping volatile components from the reaction mixture. These components were passed through a pressure valve into a condensation unit. The overall pressure in the autoclave was kept at 60 bar. To the mixture of carbon monoxide and hydrogen to be introduced methanol was continuously added at a rate of 0.4 ml/h. The total reaction time was 15 hours. During the first hour the $CO/H_2$ molar ratio of the gas mixture to be introduced was 2:1, during the next 14 hours it was 5:3. After termination of the reaction the contents both of the autoclave and of the condensation unit were analysed by gas-liquid chromatography. At each hour of the experiment samples were taken of the effluent gas stream and analysed for the presence of CO, $H_2$, $CH_4$ and $CO_2$ by gas chromatography. Methanol conversion was 93%. The product contained 19 %mol of ethyl acetate and 80 %mol of acetic acid and traces of ethanol. The reaction mixture obtained was anhydrous.

TABLE A

| Ru-compound Rh-compound (mmol) | Nitrogen compound (mmol) | Promoter (mmol) | Temp. (°C) | $CO/H_2$ molar ratio; introduction time (h) | Methanol conversion (%mol) | Selectivity (%mol) | | | |
|---|---|---|---|---|---|---|---|---|---|
| | | | | | | Ethanol | Ethyl acetate | Methyl acetate | Methane |
| $Ru(acac)_3$* 1.5 $Rh(acac)(CO)_2$ 0.25 | Pyridine oxide 20 | — | 190 | 2:1 (1 h) 5:3 (14 h) | 40 | 72 | 10 (traces ethers) | 8 | 5 |
| $Ru(acac)_3$ 0.75 $Rh(acac)(CO)_2$ 0.25 | Pyridine oxide 20 | — | 190 | 2:1 (1 h) 1:1 (14 h) | 25 | 90 | 3 | 3 | 4 |
| $Ru(acac)_3$* 0.73 $Rh(acac)(CO)_2$ 0.08 | N-methyl-pyrrolidone 78 | Triphenyl-phosphine oxide 4 | 185 | 2:1 (15 h) | 20** | 90 | 2 | 3 | 5 |
| $RuCl_3 \cdot 3H_2O$ 1.5 $RhCl_3 \cdot 3H_2O$ 0.2 | N-methyl-pyrrolidone 78 | Triphenyl-phosphine oxide 8 | 190 | 2:1 (15 h) | 23** | 91 | 2 | 2 | 5 |

*acac=acetyl acetonate
**to the $CO/H_2$ mixture was added 0.4 ml/h of additional water

Example IV

Into a magnetically stirred 125 ml Hastelloy C autoclave were placed 0.62 mol methanol (25 ml) and the quantities given in Table B of ruthenium compound, rhodium compound, alkali metal iodide, nitrogen compound and promoter. The autoclave was flushed with carbon monoxide, filled with a mixture of carbon monoxide and hydrogen of molar ratios as given in Table B, and heated to a temperature of 190°C. During the reactions mixtures of carbon monoxide and hydrogen were introduced into the autoclave at a rate of 9 Nl/h for the periods of time and in the molar ratios given in Table B, stripping volatile components from the reaction mixture. These components were passed through a pressure valve into a condensation unit. The overall pressure in the autoclave was kept at 60 bar, the temperature at 190°C and the total reaction time at 15 hours. After termination of the reaction the contents both of the autoclave and of the condensation unit were analysed by gas-liquid chromatography. At each hour of the experiment samples were taken of the effluent gas stream and analysed by gas chromatography for the presence of CO, $H_2$, $CH_4$ and $CO_2$. Methanol conversion and selectivities to the formation of ethanol, ethyl acetate, methyl acetate, acetic acid and methane are given in Table B. The reaction mixture obtained was anhydrous.

In Table C a number of comparative experiments are listed which were carried out in the same way as those of Table B. The experiments show that when an alkali metal acetate is used instead of an alkali metal iodide, or when the latter compound is omitted, the catalyst system practically loses its activity. This is also the case when instead of a weak basic reacting nitrogen compound a strong basic reacting compound is used, such as N-methylpiperidine. The use of triphenylphosphine as promoter also leads to deactivation of the catalyst system. The use of methyl iodide instead of an alkali metal iodide results only in the formation of acetic acid. When the ruthenium compound is omitted from the catalyst system, practically only acetic acid and methyl acetate and no ethanol or ethyl acetate are formed. In the last two experiments—in contrast to the experiments described in Table B and the other examples—no carbon dioxide was present in the gas mixture leaving the reactor, and the reaction mixture obtained was not anhydrous.

Example V

An experiment was carried out in the way described in Example IV using pure carbon monoxide instead of a mixture of carbon monoxide and hydrogen. In the autoclave were 0.62 mol methanol (25 ml), 0.5 mmol $RhCl_3 \cdot 3H_2O$, 0.5 mmol $RuCl_3 \cdot 3H_2O$, 40 mmol $LiI \cdot 2H_2O$ and 156 mmol N-methylpyrrolidone. The temperature was 190°C and the pressure was kept at 60 bar. Throughout the reaction period (15 hours) carbon monoxide was passed through the autoclave at a rate of 9 Nl/h. Conversion was 100%. The total reaction product contained 48 %mol ethyl acetate, 10 %mol ethanol, 20 %mol methyl acetate, 20 %mol acetic acid and traces methane and acetaldehyde.

TABLE B

| Ru-compound Rh-compound (mmol) | Alkali metal iodide (mmol) | Nitrogen compound (mmol) | Promoter (mmol) | Temp. (°C) | $CO/H_2$ molar ratio introduction time (h) | Methanol conversion (%mol) | Selectivity | | | | |
|---|---|---|---|---|---|---|---|---|---|---|---|
| | | | | | | | Ethanol | Ethyl acetate | Methyl acetate | Acetic acid | Methane |
| $RuCl_3 \cdot 3H_2O$ 1.5 | $LiI \cdot 2H_2O$ 40 | NMP* 104 | TPPO** 8 | 190 | 2:1 | 10 | 72 | 10 | 6 | — | 12 |
| $RuCl_3 \cdot 3H_2O$ 1.5 $RhCl_3 \cdot 3H_2O$ 0.4 | $LiI \cdot 2H_2O$ 30 | NMP 104 | — | 190 | 2:1 | 24 | 70 | 8 | 10 | — | 12 |
| $RuCl_3 \cdot 3H_2O$ 1.5 $RhCl_3 \cdot 3H_2O$ 0.2 | $LiI \cdot 2H_2O$ 50 | NMP 104 | TPPO 8 | 190 | 2:1 | 40 | 73 | 6 | 4 | — | 14 |
| $RuCl_3 \cdot 3H_2O$ 1.5 $RhCl_3 \cdot 3H_2O$ 0.4 | $LiI \cdot 2H_2O$ 30 | NMP 104 | TPPO 8 | 190 | 2:1 | 35 | 75 | 7 | 5 | — | 12 |

TABLE B

| Ru-compound Rh-compound (mmol) | Alkali metal iodide (mmol) | Nitrogen compound (mmol) | Promoter (mmol) | Temp. (°C) | $CO/H_2$ molar ratio introduction time (h) | Methanol conversion (%mol) | Selectivity | | | | |
|---|---|---|---|---|---|---|---|---|---|---|---|
| | | | | | | | Ethanol | Ethyl acetate | Methyl acetate | Acetic acid | Methane |
| $RuCl_3 \cdot 3H_2O$ 1.5 $RhCl_3 \cdot 3H_2O$ 0.2 | $LiI \cdot 2H_2O$*** 30 | NMP 104 | TPPO 8 | 185 | 2:1 | 40 | 72 | 9 | 5 | — | 14 |
| ditto | $LiI \cdot 2H_2O$*** 30 | NMP 104 | TPPO 8 | 190 | 2:1 | 60 | 70 | 10 | 8 | — | 12 |
| ditto | $LiI \cdot 2H_2O$*** 30 | NMP 104 | TPPO 8 | 190 | 10:1 | 90 | 40 | 30 | 15 | 5 | 10 |
| ditto | $LiI \cdot 2H_2O$ 10 | NMP 104 | TPPO 8 | 190 | 3:1 | 15 | 50 | 20 | 20 | — | 10 |
| ditto | $LiI \cdot 2H_2O$ 30 | NMP 130 | TPPO 8 | 190 | 3:1 | 15 | 70 | 10 | 5 | 10 | 5 |
| ditto | NaI 38 | NMP 104 | TPPO 8 | 190 | 2:1 (3 h) 3:1 (12 h) | 80 | 45 | 30 | 15 | — | 10 |
| ditto | NaI 38 | NMP 104 | TPPO 8 $CH_3ONa$ 1 | 190 | 2:1 | 30 | 71 | 8 | 10 | — | 10 |
| ditto | $LiI \cdot 2H_2O$ 50 | NMP 104 | tributyl-phosphine oxide 8 | 190 | 2:1 | 60 | 74 | 4 | 2 +propanol (5) | — | 10 |

*NMP=N-methylpyrrolidone
**TPPO=Triphenylphosphine oxide
***to the reaction mixture was added 7 ml trimethylorthoformate in order to remove water from $LiI \cdot 2H_2O$

TABLE C
(comparative experiments)

| Ru-compound Rh-compound (mmol) | Alkali metal iodide (mmol) | Nitrogen compound (mmol) | Promoter (mmol) | Temp. (°C) | $CO/H_2$ molar ratio | Methanol conversion (%mol) | Selectivity | | | | |
|---|---|---|---|---|---|---|---|---|---|---|---|
| | | | | | | | Ethanol | Ethyl acetate | Methyl acetate | Acetic acid | Methane |
| $RuCl_3 \cdot 3H_2O$ 1.5 $RhCl_3 \cdot 3H_2O$ 0.2 | — | NMP 104 | TPPO 8 | 190 | 3:1 | — | — | — | — | — | — |
| ditto | $Li(CH_3COO) \cdot 2H_2O$ 30 | NMP 104 | TPPO 8 | 190 | 3:1 | <3 | 30 | — (methyl formate 60) | — | — | 10 |
| ditto | $LiI \cdot 2H_2O$ 40 | N-methyl-piperidine 82 | — | 190 | 3:1 | <3 | — | — | — | — | — |
| ditto | $LiI \cdot 2H_2O$ 40 | NMP 104 | TPP* 8 | 190 | 2:1 | 4.5 | 45 | 10 | 35 | — | 10 |
| ditto | $CH_3I$ 40 | NMP 104 | TBPO** 8 | 190 | 2:1 | 100 | — | traces (traces ethers) | — | 96 | traces |
| $RhCl_3 \cdot 3H_2O$ 0.5 | $LiI \cdot 2H_2O$ 40 | NMP 104 | TPPO 8 | 190 | 2:1 | 60 | — | traces (traces acetaldehyde) | 65 | 33 | — |

*TPP=triphenylphosphine
**TBPO=tributylphosphine oxide

**Claims**

1. A process for the preparation of ethanol and/or ethyl acetate by reaction of methanol with carbon monoxide or a mixture of carbon monoxide and hydrogen in the presence of a catalyst system containing a ruthenium compound and an organic nitrogen compound, characterized in that an iodide and/or bromide of an alkali metal or an alkaline earth metal is present in the reaction mixture and that the organic nitrogen compound comprises an aromatic heterocyclic amine or an oxide thereof or contains a group

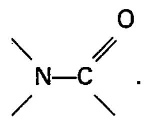 .

2. A process as claimed in claim 1, characterized in that the catalyst system comprises a rhodium compound.

3. A process as claimed in claim 2, characterized in that the ratio of the number of gram atoms ruthenium to the number of gram atoms rhodium lies between 50:1 and 1:5.

4. A process as claimed in claim 3, characterized in that the ratio of the number of gram atoms ruthenium to the number of gram atoms rhodium lies between 20:1 and 1:2.

5. A process as claimed in any one of the preceding claims, characterized in that lithium iodide or sodium iodide is used.

6. A process as claimed in any one of the preceding claims, characterized in that the ratio of the number of gram atoms I plus Br to the number of gram atoms Ru plus Rh lies between 3:1 and 200:1.

7. A process as claimed in claim 6, characterized in that the ratio of the number of gram atoms I plus Br to the number of gram atoms Ru plus Rh lies between 4:1 and 150:1.

8. A process as claimed in any one of the preceding claims, characterized in that the organic nitrogen compound is substituted with pyridine, chinoline or isochinoline or an oxide of pyridine, chinoline or isochinoline.

9. A process as claimed in any one of the preceding claims, characterized in that the organic nitrogen compound is a picoline or an oxide thereof.

10. A process as claimed in any one of claims 1—6, characterized in that the organic nitrogen compound is an amide, a carbamate, urea or a urea derivative.

11. A process as claimed in claim 10, characterized in that the amide has the formula:

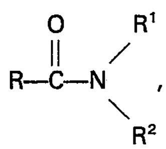 ,

wherein R represents a hydrogen atom or an alkyl, cycloalkyl, aryl, alkaryl or aralkyl group having not more than 10 carbon atoms, $R^1$ and $R^2$ independently represent a hydrogen atom or an alkyl, aryl, alkaryl or aralkyl group which may contain a

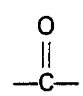

group, and has not more than 10 carbon atoms, or $R^1$ and $R^2$, together with the nitrogen atom to which they are bound, form a cyclic group which has not more than 5 carbon atoms and which may contain one or more nitrogen or oxygen atoms, or one of the groups $R^1$ and $R^2$, together with the nitrogen atom and R, forms a cyclic group having not more than 5 carbon atoms.

12. A process as claimed in claim 11, characterized in that the amide is N,N-dimethylacetamide or N-methylpyrrolidone.

13. A process as claimed in claim 10, characterized in that the carbamate has the formula:

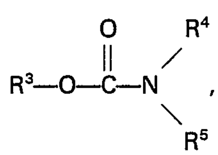 ,

wherein $R^3$ represents an alkyl, aryl, aralkyl or alkaryl group having not more than 10 carbon atoms and $R^4$ and $R^5$ independently represent a hydrogen atom or an alkyl, aryl, aralkyl or alkaryl group having not more than 10 carbon atoms.

14. A process as claimed in claim 10, characterized in that the urea (derivative) has the formula:

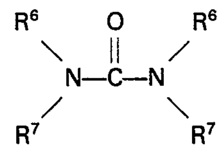

wherein $R^6$ and $R^7$ independently represent a hydrogen atom or an alkyl, aryl, aralkyl or alkaryl group having not more than 10 carbon atoms.

15. A process as claimed in any one of the preceding claims, characterized in that the ratio of the number of moles of the organic nitrogen compound to the number of gram atoms Ru plus Rh lies between 5:1 and 200:1.

16. A process as claimed in claim 15, characterized in that when the organic nitrogen compound contains a group

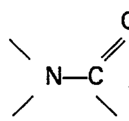,

said ratio lies between 30:1 and 200:1 and when the organic nitrogen compound is an aromatic heterocyclic amine or an oxide thereof, said ratio lies between 5:1 and 50:1.

17. A process as claimed in any one of the preceding claims, characterized in that the reaction is carried out in the presence of an oxide of a secondary or tertiary phosphine or a basic compound of an alkali or alkaline earth metal as promoter.

**Patentansprüche**

1. Ein Verfahren zur Herstellung von Äthanol und/oder Äthylacetat durch Umsetzung von Methanol mit Kohlenmonoxid oder einer Mischung aus Kohlenmonoxid und Wasserstoff in Gegenwart eines Katalysatorsystems enthaltend eine Rutheniumverbindung und eine organische Stickstoffverbindung, dadurch gekennzeichnet, daß ein Jodid und/oder Bromid eines Alkalimetalls oder Erdalkalimetalls in der Reaktionsmischung vorliegt und daß die organische Stickstoffverbindung ein aromatisches heterocyclisches Amin oder ein Oxid davon ist, oder eine Gruppe

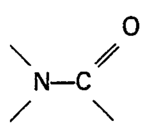

enthält.

2. Ein Verfahren wie in Anspruch 1 beansprucht, dadurch gekennzeichnet, daß das Katalysatorsystem eine Rhodiumverbindung enthält.

3. Ein Verfahren wie in Anspruch 2 beansprucht, dadurch gekennzeichnet, daß das Verhältnis der Zahl der Grammatome Ruthenium zur Zahl der Grammatome Rhodium in Bereich zwischen 50:1 und 1:5 liegt.

4. Ein Verfahren wie in Anspruch 3 beansprucht, dadurch gekennzeichnet, daß das Verhältnis der Zahl der Grammatome Ruthenium zur Zahl der Grammatome Rhodium im Bereich zwischen 20:1 und 1:2 liegt.

5. Ein Verfahren wie in einem der vorstehenden Ansprüche beansprucht, dadurch gekennzeichnet, daß Lithiumiodid oder Natriumiodid verwendet wird.

6. Ein Verfahren wie in einem der vorstehenden Ansprüche beansprucht, dadurch gekennzeichnet, daß das Verhältnis der Zahl der Grammatome Jod plus der Zahl der Grammatome Brom zur Zahl der Grammatome Ruthenium plus der Zahl der Grammatome Rhodium im Bereich zwischen 3:1 und 200:1 liegt.

7. Ein Verfahren wie in Anspruch 6 beansprucht, dadurch gekennzeichnet, daß das Verhältnis der Zahl der Grammatome Jod plus der Zahl der Grammatome Brom zur Zahl der Grammatome Ruthenium plus der Zahl der Grammatome Rhodium im Bereich von 4:1 bis 150:1 liegt.

8. Ein Verfahren wie in einem der vorstehenden Ansprüche beansprucht, dadurch gekennzeichnet, daß die organische Stickstoffverbindung durch Pyridin, Chinolin oder Isochinolin oder ein Oxid von Pyridin, Chinolin oder Isochinolin substituiert ist.

9. Ein Verfahren wie in einem der vorstehenden Ansprüche beansprucht, dadurch gekennzeichnet, daß die organische Stickstoffverbindung ein Picolin oder ein Oxid desselben ist.

10. Ein Verfahren wie in einem der Ansprüche 1 bis 6 beansprucht, dadurch gekennzeichnet, daß die organische Stickstoffverbindung ein Amid, ein Carbamat, Harnstoff oder ein Harnstoffderivat ist.

11. Ein Verfahren wie in Anspruch 10 beansprucht, dadurch gekennzeichnet, daß das Amid die Formel

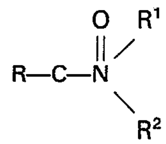

hat, wobei R ein Wasserstoffatom oder eine Alkyl-, Cycloalkyl-, Aryl-, Alkaryl-oder Aralkylgruppe mit nicht mehr als 10 Kohlenstoffatomen ist, $R^1$ und $R^2$ unabhängig voneinander ein Wasserstoffatom oder eine Alkyl-, Aryl-, Alkaryl- oder Aralkylgruppe sind, welche eine

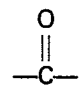

Gruppe enthalten können und nicht mehr als 10 Kohlenstoffatome aufweisen, oder $R^1$ und $R^2$ zusammen mit dem Stickstoffatom, an das sie gebunden sind, eine cycliscne Gruppebilden, welche nicht mehr als 5 Kohlenstoffatome aufweist und welche 1 oder mehrere Stickstoffatom(e) oder Sauerstoffatom(e) aufweisen kann, oder eine der Gruppen $R^1$ und $R^2$ zusammen mit dem Stickstoffatom und R, eine cyclische Gruppe bildet, welche nicht mehr als 5 Kohlenstoffatome aufweist.

12. Ein Verfahren wie in Anspruch 11 beansprucht, dadurch gekennzeichnet, daß das Amid N,N-Dimethylacetamid oder N-Methylpyrrolidon ist.

13. Ein Verfahren wie in Anspruch 10 beansprucht, dadurch gekennzeichnet, daß das Carbamat die Formel

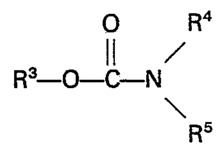

hat, in welcher $R^3$ eine Alkyl-, Aryl-, Aralkyl- oder Alkarylgruppe bedeutet, welche nicht mehr als 10 Kohlenstoffatome aufweist, und $R^4$ und $R^5$ unabhängig voneinander ein Wasserstoffatom oder eine Alkyl-, Aryl-, Aralkyl- oder Alkarylgruppe mit nicht mehr als 10 Kohlenstoffatomen darstellen.

14. Ein Verfahren wie in Anspruch 10 beansprucht, dadurch gekennzeichnet, daß der (das) Harnstoff (Derivat) die Formel:

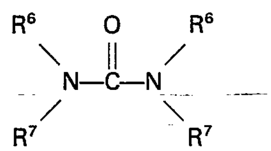

hat, in welcher $R^6$ und $R^7$ unabhängig voneinander ein Wasserstoff-atom oder eine Alkyl-, Aryl-, Aralkyl- oder Alkarylgruppe mit nicht mehr als 10 Kohlenstoffatomen sind.

15. Ein Verfahren wie in einem der vorstehenden Ansprüche beansprucht, dadurch gekennzeichnet, daß das Verhältnis der Zahl der Mole der organischen Stickstoffverbindung zur Zahl der Grammatome Ruthenium plus der Zahl der Grammatome Rhodium im Bereich zwischen 5:1 und 200:1 liegt.

16. Ein Verfahren wie in Anspruch 15 beansprucht, dadurch gekennzeichnet, daß, wenn die organische Stickstoffverbindung eine Gruppe

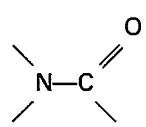

enthält, das genannte Verhältnis im Bereich zwischen 30:1 und 200:1 liegt und, wenn die organische Stickstoffverbindung ein aromatisches heterocyclisches Amin oder ein Oxid davon ist, das genannte Verhältnis im Bereich zwischen 5:1 und 50:1 liegt.

17. Ein Verfahren wie in einem der vorstehenden Ansprüche beansprucht, dadurch gekennzeichnet, daß die Umsetzung in Gegenwart eines Oxids eines sekundären oder tertiären Phosphins oder einer basischen Verbindung eines Alkali- oder Erkalkalimetalls als Promotor durchgeführt wird.

**Revendications**

1. Procédé de préparation d'éthanol et/ou d'acétate d'éthyle par réaction du méthanol avec l'oxyde de

carbone ou un mélange d'oxyde de carbone et d'hydrogène en présence d'un système de catalyseur contenant un composé au ruthénium et un composé organique de l'azote, caractérisé en ce qu'il y a un iodure et/ou un bromure d'un métal alcalin ou d'un métal alcalino-terreux présent dans le mélange de réaction, et en ce que le composé organique de l'azote se compose d'une amine aromatique hétérocyclique ou d'un oxyde de celle-ci ou contient un groupe

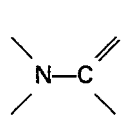 .

2. Procédé selon la revendication 1, caractérisé en ce que le système de catalyseur comprend un composé au rhodium.

3. Procédé selon la revendication 2, caractérisé en ce que le rapport du nombre d'atomes—grammes de ruthénium au nombre d'atomes—grammes de rhodium est compris entre 50:1 et 1:5.

4. Procédé selon la revendication 3, caractérisé en ce que le rapport du nombre d'atomes—grammes de ruthénium au nombre d'atomes—grammes de rhodium est compris entre 20:1 et 1:2.

5. Procédé selon l'une quelconque des revendications précédentes, caractérisé en ce qu'on utilise l'iodure de lithium ou l'iodure de sodium.

6. Procédé selon l'une quelconque des revendications précédentes, caractérisé en ce que le rapport du nombre d'atomes—grammes d'I plus Br au nombre d'atomes—grammes de Ru plus Rh est compris entre 3:1 et 200:1.

7. Procédé selon la revendication 6, caractérisé en ce que le rapport du nombre d'atomes—grammes d'I plus Br au nombre d'atomes—grammes de Ru plus Rh est compris entre 4:1 et 150:1.

8. Procédé selon l'une quelconque des revendications précédentes caractérisé en ce que le composé organique de l'azote est représenté par la pyridine, la quinoléine ou l'isoquinoléine éventuellement substituée ou un oxyde de la pyridine, de la quinoléine ou de l'isoquinoléine éventuellement substituée.

9. Procédé selon l'une quelconque des revendications précédentes, caractérisé en ce que le composé organique de l'azote est une picoline ou un oxyde de celle-ci.

10. Procédé selon l'une quelconque des revendications 1 à 6, caractérisé en ce que le composé organique de l'azote est un amide, un carbamate, l'urée ou un dérivé de l'urée.

11. Procédé selon la revendication 10, caractérisé en que l'amide a la formule:

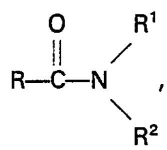 ,

dans laquelle R représente un atome d'hydrogène ou un groupe alkyle, cycloalkyle, aryle, alkaryle ou aralkyle n'ayant pas plus de 10 atomes de carbone, $R^1$ et $R^2$ représentent indépendamment un atome d'hydrogène ou un groupe alkyle, aryle, alkaryle ou aralkyle qui peut contenir un groupe

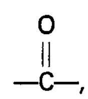

et qui n'a pas plus de 10 atomes de carbone, ou $R^1$ et $R^2$, conjointement avec l'atome d'azote auquel ils sont liés, forment un groupe cyclique qui n'a pas plus de 5 atomes de carbone et qui peut contenir un ou plusieurs atomes d'azote ou d'oxygène, ou un des groupes $R^1$ et $R^2$ conjointement avec l'atome d'azote et R, forme un groupe cyclique n'ayant pas plus de 5 atomes de carbone.

12. Procédé selon la revendication 11, caractérisé en ce que l'amide est le N,N-diméthylacétamide ou la N-méthylpyrrolidone.

13. Procédé selon la revendication 10, caractérisé en ce que le carbamate a la formule

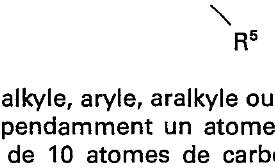 ,

dans laquelle $R^3$ représente un groupe alkyle, aryle, aralkyle ou alkaryle n'ayant pas plus de 10 atomes de carbone et $R^4$ et $R^5$ représentent indépendamment un atome d'hydrogène ou un groupe alkyle, aryle, aralkyle ou alkaryle n'ayant pas plus de 10 atomes de carbone.

14. Procédé selon la revendication 10, caractérisé en ce que l'urée (et ses dérivés) à la formule:

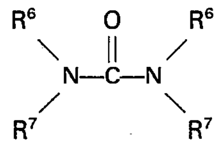

dans laquelle $R^6$ et $R^7$ représentent indépendamment un atome d'hydrogène ou un groupe alkyle, aryle, aralkyle ou alkaryle n'ayant pas plus de 10 atomes de carbone.

15. Procédé selon l'une quelconque des revendications précédentes, caractérisé en ce que le rapport du nombre de moles du composé organique de l'azote au nombre d'atomes—grammes de Ru plus Rh est compris entre 5:1 et 200:1.

16. Procédé selon la revendication 15, caractérisé en ce que lorsque le composé organique de l'azote contient un groupe

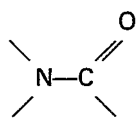

ledit rapport est compris entre 30:1 et 200:1, et en ce que lorsque le composé organique de l'azote est une amine aromatique hétérocyclique ou un oxyde de celle-ci, ledit rapport est compris entre 5:1 et 50:1.

17. Procédé selon l'une quelconque des revendications précédentes, caractérisé en ce que la réaction s'éffectue en présence d'un oxyde d'une phosphine secondaire ou tertiaire ou d'un composé basique d'un métal alcalin ou alcalino-terreux à titre de promoteur.